# EUROPÄISCHE PATENTSCHRIFT

(11) **EP 0 561 126 B1**
(45) Veröffentlichungstag und Bekanntmachung des Hinweises auf die Patenterteilung: **22.05.1996**
(21) Anmeldenummer: 93101301.5
(22) Anmeldetag: 28.01.1993
(51) Int. Cl.: A61B 5/00, G01N 21/47

(54) **Kalibrierreflektorvorrichtung für ein optisches Messsystem**
Calibration reflector device for an optical measuring system
Dispositif de calibration à réflecteur pour un système de mesure optique

(30) Priorität: 18.03.1992 DE 4208707
(43) Veröffentlichungstag der Anmeldung: 22.09.1993
(73) Patentinhaber: PULSION Verwaltungs GmbH & Co Medizintechnik KG, D-81675 München (DE)
(72) Erfinder: Pfeiffer, Ulrich, Dr., D-81667 München (DE); Knoll, Reinhold, D-81675 München (DE)
(74) Vertreter: WILHELMS, KILIAN & PARTNER Patentanwälte

(56) Entgegenhaltungen:
- EP-A- 0 271 304
- EP-A- 0 366 840
- EP-A- 0 397 496

## Beschreibung

Die Erfindung betrifft eine Kalibrierreflektorvorrichtung für ein optisches Meßsystem nach dem Gattungsbegriff des Patentanspruchs 1. Eine derartige Kalibrierreflektorvorrichtung ist aus der US PS 4 796 633 bekannt.

Kalibrierreflektorvorrichtungen werden zum Eichen von optischen Meßsystemen verwandt, die dazu dienen, Parameter einer Probe zu messen, die sich über spektroskopische Unterschiede in der Probe erfassen lassen. Dazu zählen beispielsweise Kathetersauerstoffmeßsysteme, die dazu verwandt werden, die Sauerstoffsättigung des Blutes zu messen. Zur Messung der Sauerstoffsättigung des Blutes in vivo wird die Lichtleitersonde im Kreislauf des Patienten angeordnet.

Bei derartigen optischen Meßsystemen besteht die Lichtleitersonde beispielsweise aus einem dünnen Schlauch mit zwei Lichtwellenleitern, deren meßseitiges Ende senkrecht abgeschnitten und poliert ist. Das von einer Lichtquelle kommende, durch einen Lichtwellenleiter hindurchgehende Licht trifft bei der Messung auf die zu messende Probe und wird durch die Probe gestreut. Das gestreute Licht wird wieder aufgenommen und zur Messung ausgewertet. Die Messungen erfolgen mit Licht verschiedener Wellenlängen, wobei beispielsweise die sauerstoffsättigung des Blutes durch eine Verhältnisbildung der Meßwerte bei verschiedenen Wellenlängen ermittelt wird.

Optische Meßsysteme dieser Art müssen geeicht werden, da die Systeme mit der Zeit altern und ein Meßwertdrift auftritt. Der Grund dafür liegt z.B. darin, daß sich Wasser im Kunststoffmaterial der Lichtwellenleiter einlagert, daß Transmissionsänderungen auftreten, daß sich die Reproduzierbarkeit der Steckverbindung zwischen der Lichtleitersonde und der anzuschließenden Lichtquelle bzw. dem anzuschließenden Auswertegerät ändert oder daß die lichtaussendenden Dioden der Lichtquelle altern.

Ohne eine Eichung des optischen Meßsystems können keine absoluten sondern nur relative Werte gemessen werden. Eine Messung von absoluten Werten setzt ein Nachjustieren des optischen Meßsystems auf einen Standard voraus. Dazu dienen Kalibrierreflektorvorrichtungen, die einen derartigen Standard darstellen und vor der eigentlichen Messung zum Eichen des optischen Meßsystems verwandt werden. Zu diesem Zweck wird die optische Lichtleitersonde in der Kalibrierreflektorvorrichtung angeordnet, die ein bestimmtes festes Reflektionsverhalten zeigt und wird das Meßsystem über das erhaltene Meßergebnis beispielsweise durch Justieren der erhaltenen Meßsignalamplitude am Meßgerät geeicht. Nach dem Eichen mit einer Kalibrierreflektorvorrichtung, die nur einmal verwandt wird, kann die eigentliche Messung erfolgen.

Kalibrierreflektorvorrichtungen üblicher Bauweise lassen sich in sogenannte Festkörperreflektoren und sogenannte Hohlraumreflektoren unterteilen.

Ein Beispiel eines bekannten Hohlraumreflektors ist die eingangs genannte Kalibrierreflektorvorrichtung, die aus der US PS 4 796 633 bekannt ist. Bei dieser bekannten Vorrichtung sind reflektierende Teilchen an der Stirnseite in die Innenwand des Gehäuses eingebettet und wird das vordere Ende der Lichtleitersonde dieser Stirnwand in einem gewissen Abstand gegenüber angeordnet. Zur Eichung wird Licht mit bestimmten Wellenlängen durch die Lichtleitersonde auf die mit den streuenden Partikeln versehene reflektierende Stirnfläche geworfen und wird das davon reflektierte Licht aufgenommen und zur Eichung verwandt.

Bei einem derartigen Hohlrraumreflektor werden jedoch die optischen Eigenschaften durch die Oberflächenbeschaffenheit der Hohlraumwände, d.h. der Innenwände des Gehäuses, die Oberflächenbeschaffenheit der Lichtaustrittsfläche am vorderen Ende der Lichtleitersonde und die genaue Positionierung der Lichtleitersonde im Gehäuse beeinflußt. Da derartige Reflektoren einteilig im Spritzgußverfahren hergestellt werden, werden die optischen Eigenschaften darüberhinaus durch die Verteilung, Form und Größe der reflektierenden Partikeln beeinflußt, die in die Gehäusewand eingebettet sind.

Ein Beispiel eines bekannten Festkörperreflektors ist der US PS 4 322 164 zu entnehmen. Bei diesem Festkörperreflektor ist ein fester Körper im Inneren eines Gehäuses vorgesehen, in den lichtstreuende Teilchen eingebettet sind und eine Trübung verursachen, so daß dieser Festkörper ein bestimmtes bekanntes Reflektionsverhalten hat und den Meßstandard bildet. Um einen sicheren Kontakt zwischen der Oberfläche dieses Festkörpers und dem vorderen Ende der Lichtleitersonde zu gewährleisten, ist der Festkörper in axialer Richtung des Gehäuses, d.h. in axialer Richtung der Lichtleitersonde nachgiebig angeordnet und an der der Lichtleitersonde gegenüberliegenden Seite mit einer Vorspanneinrichtung - beispielsweise in Form einer Feder - versehen, über die der Festkörper bei der Eichung fest gegen das vordere Ende der Lichtleitersonde gedrückt wird. Auch bei derartigen Festkörperreflektoren werden die optischen Eigenschaften des Reflektors durch die Verteilung, Form und Größe der reflektierenden Partikeln im Festkörper beeinflußt. Diese Parameter lassen sich bei der Herstellung des Reflektors schlecht steuern. Die reflektierenden Partikeln an der Oberfläche des Festkörpers stehen aufgrund der Federvorspannung in einem ständigen festen Kontakt mit dem vorderen Ende der Lichtleitersonde, so daß die Gefahr besteht, daß sich die Teilchen vom Festkörper lösen. Darüberhinaus ist es nicht ohne weiteres möglich, die Partikelart zu ändern, da in der Regel eine Änderung des Fertigungsverfahrens erforderlich ist, so daß die Variabilität des Reflektionsverhaltens bei derartigen Reflektoren gering ist.

Bei dem in der US PS 4 322 164 beschriebenen Festkörperreflektor können zwar störende Reflektionen - insbesondere an der Ankoppelfläche des vorderen Endes der Lichtleitersonde - vermieden werden, die beispielsweise bei Hohlraumreflektoren auftreten; durch die auftretende Deformation beim festen Andrücken des Festkörpers gegen das vordere Ende der Lichtleitersonde wird jedoch die Partikelverteilung zumindest gestört.

Die der Erfindung zugrundeliegende Aufgabe besteht demgegenüber darin, die Kalibrierreflektorvorrichtung nach dem Gattungsbegriff des Patentanspruchs 1 so auszubilden, daß sie eine höhere Genauigkeit hat, d.h. geringeren Störungen ausgesetzt ist.

Diese Aufgabe wird gemäß der Erfindung durch die Ausbildung gelöst, die im Kennzeichen des Patentanspruchs 1 angegeben ist.

Bei der erfindungsgemäßen Kalibrierreflektorvorrichtung wird die Güte der Kalibrierung vorwiegend durch die transparente Scheibe und die reflektierende Einrichtung allein bestimmt, während die anderen Bauteile keinen störenden Einfluß ausüben können und somit nach beliebigen Verfahren hergestellt werden können. Auf Grund der transparenten Zwischenschicht befinden sich alle Reflektionszentren etwa in gleicher Entfernung vom vorderen Ende der optischen Lichtleitersonde, was zur Folge hat, daß Größe, Form und Dichte der reflektierenden Partikeln der reflektierenden Einrichtung einen geringen Einfluß auf die Güte der Kalibrierung haben.

Ein weiterer wesentlicher Vorteil der erfindungsgemäßen Ausbildung ist die Möglichkeit der Steuerung des Reflektionsverhaltens auf Grund des größeren Spielraumes bei der Wahl der reflektierenden Partikeln. Durch Anpassung des Brechungsindizes von Lichtwellenleiter und transparenter Scheibe können störende Reflektionen an der Lichtaustrittsfläche der Lichtleiter vermieden werden.

Die erfindungsgemäße Kalibrierreflektorvorrichtung zeigt somit eine gute Reproduzierbarkeit bei der Fertigung und geringe Störungen der optischen Eigenschaften durch Nebeneffekte wie beispielsweise störende Reflektionen an der Ankoppelfläche der optischen Lichtleitersonde gegenüber der reflektierenden Einrichtung.

Besonders bevorzugte Weiterbildungen und Ausgestaltungen der erfindungsgemäßen Kalibrierreflektorvorrichtung sind Gegenstand der Patentansprüche 2-7.

Im folgenden wird anhand der zugehörigen Zeichnung ein besonders bevorzugtes Ausführungsbeispiel der Erfindung näher beschrieben. Es zeigen
Fig. 1 eine Schnittansicht des Ausführungsbeispiels der erfindungsgemäßen Kalibrierreflektorvorrichtung mit teileingeschobener Lichtleitersonde,
Fig. 2 eine Schnittansicht der erfindungsgemäßen Kalibrierreflektorvorrichtung mit vollständig eingeschobener Lichtleitersonde und
Fig. 3 in einer Teilschnittansicht den optisch aktiven Bereich des Ausführungsbeispiels der erfindungsgemäßen Kalibrierreflektorvorrichtung im einzelnen.

Das in der Zeichnung dargestellte Ausführungsbeispiel der erfindungsgemäßen Kalibrierreflektorvorrichtung umfaßt im wesentlichen ein Gehäuse 12 - beispielsweise aus einem lichtundurchlässigen Kuststoffmaterial -, das langgestreckt ausgebildet und an einer Seite offen ist.

Im Inneren des Gehäuses 12 sind eine reflektierende Scheibe oder Schicht bzw. opake Schicht 14 sowie eine transparente Schicht oder Scheibe 13 in axialer Richtung in dieser Reihenfolge vorgesehen.

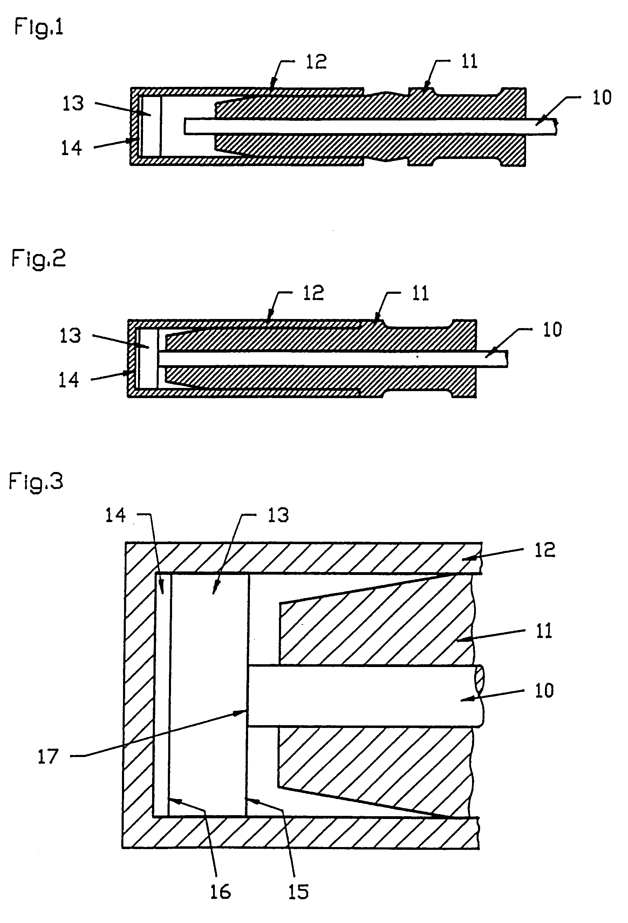

Wie es in Fig. 3 dargestellt ist, besteht der optisch aktive Bereich somit aus der transparenten Scheibe 13 und der opaken Schicht 14, die ohne Zwischenraum fest miteinander verbunden sind. Die transparente Scheibe 13 wird von einer Ankopplungsfläche 15 und einer reflektierenden Fläche 16, d.h. der Grenzfläche zur opaken Schicht 14 begrenzt. Die Form der Ankopplungsfläche 15 ist an die Lichtleitersonde oder Meßsonde 10 angepaßt, wobei gewöhnlich eine plane Ankopplungsfläche 15 vorgesehen ist.

Die opake Schicht 14, die die reflektierende Einrichtung darstellt, kann aus einer Beschichtung der transparenten Schicht 13 oder aus einer dünnen Scheibe bestehen, in die kunstharzgebundene Pigmente eingebunden sind. Die Beschichtung kann beispielsweise im Siebdruckverfahren auf die transparente Scheibe 13 aufgebracht sein. Die opake Schicht 14 kann jedoch auch durch die Stirnfläche des Gehäuses gebildet sein, indem in diese Stirnfläche Pigmente eingebracht sind. In diesem Fall ist die transparente Scheibe 13 ohne Zwischenraum an der Stirnfläche des Gehäuses 12 angeordnet.

Die transparente Scheibe 13 und gegebenenfalls die opake Scheibe 14 werden vorzugsweise lose in das Gehäuse 12 eingepreßt. Die zur Bildung der reflektierenden Einrichtung, d.h. der opaken Schicht oder Scheibe 14 verwandte Pigmentmischung hat ein bekanntes bestimmtem Mischverhältnis, das problemlos geändert werden kann, um die Reflektionseigenschaften zu variieren.

Die transparente Scheibe 13 ist lichtdurchlässig, im idealen Fall ohne Absorption und besteht beispielsweise aus einem elastischen, lichtdurchlässigen Kunststoff - wie beispielsweise einem Silikon.

Die reflektierende Fläche 16 gemäß Fig. 3 kann auch durch eine rauhe, teilweise totalreflektierende Grenzfläche gebildet sein, wobei in diesem Fall die opake Schicht 14 durch eine Schicht mit niedrigerem Brechungsindex ersetzt ist. Diese kann auch eine Luftschicht sein.

Ein Haltestopfen 11 aus einem elastischen Material ist mit einer zentralen Bohrung zur Aufnahme des vorderen Endes der Lichtleitersonde 10 versehen. Der Durchmesser dieser Bohrung ist so gestaltet, daß die Lichtleitersonde 10 darin mit einem gewissen Reibungswiderstand aufgenommen ist. Ansätze oder Vorsprünge am Stopfen 11 in einem gewissen axialen Abstand von seinem vorderen Ende sind so angeordnet und ausgebildet, daß beim vollständigen Einschieben des Stopfens 11 gemäß Fig. 2 das vordere Ende der Lichtleitersonde an der Oberfläche der transparenten Scheibe 13 ohne Zwischenraum anliegt. Die Scheibe 13 sorgt somit für einen Abstand zwischen dem vorderen Ende der Lichtleitersonde 10 und der opaken Schicht 14, d.h. der reflektierenden Einrichtung.

Zur Abschirmung von Fremdlicht bestehen das Gehäuse 12 und der Haltestopfen 11 vorzugsweise aus einem lichtundurchlässigen Material.

Das oben beschriebene Ausführungsbeispiel der erfindungsgemäßen Kalibrierreflektorvorrichtung wird in der folgenden Weise benutzt:
Die Reflektorvorrichtung wird bei der Herstellung des Meßsystems, d.h. der optischen Lichtleitersonde 10 im betriebsbereiten Zustand auf die Sonde 10 aufgebracht und geprüft. Die Sonde 10 und der Reflektor bleiben bis zur Benutzung miteinander verbunden. Unmittelbar vor dem Gebrauch wird die Sonde mit dem Reflektor als Standard vom Benutzer geprüft und wird das zugehörige Meßsystem geeicht.

Zur Montage wird die faseroptische Lichtleitersonde 10 in den Haltestopfen 11 gesteckt. Die Sonde 10 wird in einem gewissen Maße durch den Haltestopfen 11 hindurchgeschoben, bis ihr Ende aus dem Haltestopfen 11 vorsteht. Anschließend wird der Haltestopfen 11 mit der Sonde 10 in das Gehäuse 12 eingeschoben. Durch die Verformung des Haltestopfens 11 wird die Sonde 10 festgeklemmt. Wenn die Stirnfläche der Sonde 10 mit der Ankopplungsfläche 15 der transparenten Scheibe 13 in Berührung kommt, wird die Sonde 10 gegen die Reibungskraft im Haltestopfen 11 zurückgeschoben. In dieser Weise wird sichergestellt, daß das vordere Ende der Sonde 10 fest an der Ankopplungsfläche 15 der transparenten Schicht 13 anliegt.

Die Haftreibung zwischen dem Gehäuse 12, dem Haltestopfen 11 und der Sonde 10 führt zu einer dauerhaften Festlegung der Sonde 10 in ihrer Position. Durch die elastische Verformung des Haltestopfens 11 bleibt die Sonde 10 auch nach der Montage an der Ankopplungsfläche 15. In Verbindung mit der Elastizität der transparenten Scheibe 13 wird dadurch eine reflektionsfreie Lichtankoplung erreicht.

Zur Eichung der Sonde 10 wird Licht von der Sonde 10 über die Ankopplungsfläche 15 in die transparente Scheibe 13 gestrahlt. Das Licht trifft auf die reflektierende Fläche 16 und wird von dieser diffus und wellenlängenselektiv reflektiert. Ein Teil des reflektierten Lichtes wird in die Sonde 10 zurückgeworfen und kann zur Eichung des Meßsystems ausgewertet werden.

Durch eine Variation der Dicke der transparenten Scheibe 13 und der reflektierenden Fläche 16 bzw. der reflektierenden Schicht 14 kann das optische Verhalten verändert werden.

Zur eigentlichen Messung wird der Haltestopfen 11 anschließend aus dem Gehäuse 12 herausgezogen, wodurch sich die ursprüngliche Form des Haltestopfens 11 zurückbildet. Die Meßsonde 10 wird dadurch entlastet und kann aus dem Haltestopfen 12 herausgezogen und zur eigentlichen Messung eingesetzt werden.

Bei der erfindungsgemäßen Kalibrierreflektorvorrichtung läßt sich bei gleichem Herstellungsverfahren das Reflektionsverhalten in einfacher Weise anpassen. Das kann beispielsweise durch eine Änderung der Dicke der transparenten Schicht 13 erreicht werden. Das spektrale Verhalten kann über die Pigmentierung der opaken Schicht 14 verändert werden. Die transparente Scheibe 13 kann aus einem Plattenmaterial gestanzt werden, wobei die opake Schicht 14 im Siebdruckverfahren vorher aufgebracht werden kann. Daraus ergibt sich eine preiswerte, reproduzierbare Fertigung.

Die Qualität der Eichung wird vorwiegend durch die transparente Scheibe 13 und die opake Schicht 14 bestimmt. Das hat zur Folge, daß alle anderen Bauteile nach beliebigen Verfahren hergestellt werden können, da sie keine Störeinflüsse ausüben können.

Besondere Anforderungen an die Konstruktion des Katheters für den Einsatz in vitro zur Messung der Sauerstoffsättigung im Blut werden nicht gestellt.

Durch die paßgenaue, im Brechungsindex angepaßte Ankopplung der optischen Lichtleitersonde an den Reflektionsstandard werden Störreflektionen vermieden.

## Patentansprüche

1. Kalibrierreflektorvorrichtung für ein optisches Meßsystem, das eine optische Lichtleitersonde (10) aufweist, mit
- einem an einer Seite offenen Gehäuse (12),
- einer reflektierenden Einrichtung (14) mit einem bestimmten Reflektionsverhalten und
- einer Positioniereinrichtung (11), die das vordere Ende der Lichtleitersonde im Gehäuse der reflektierenden Einrichtung gegenüber anordnet, gekennzeichnet durch eine transparente Scheibe (13), die vor der reflektierenden Einrichtung (14) angeordnet ist, derart, daß das vordere Ende der Lichtleitersonde (10) von der Positioniereinrichtung (11) an der transparenten Scheibe (13) anliegend angeordnet wird.

2. Kalibrierreflektorvorrichtung nach Anspruch 1 dadurch gekennzeichnet, daß die reflektierende Einrichtung (14) aus einer reflektierenden Schicht besteht, in der reflektierende Partikeln angeordnet sind.

3. Kalibrierreflektorvorrichtung nach Anspruch 2 dadurch gekennzeichnet, daß die reflektierende Schicht auf die transparente Scheibe (13) geschichtet ist.

4. Kalibrierreflektorvorrichtung nach Anspruch 1 dadurch gekennzeichnet, daß die reflektierende Einrichtung dadurch gebildet ist, daß in der Stirnwand des Gehäuses (12) reflektierende Partikeln eingebettet sind.

5. Kalibrierreflektorvorrichtung nach Anspruch 1 dadurch gekennzeichnet, daß die an die reflektierende Einrichtung (14) grenzende Oberfläche (16) der transparenten Scheibe (13) in Form einer rauhen, teilweise totalreflektierenden Grenzfläche ausgebildet ist und die reflektierende Einrichtung (14) aus einer opaken Schicht mit niedrigerem Brechungsindex besteht.

6. Kalibrierreflektorvorrichtung nach einem der vorhergehenden Ansprüche dadurch gekennzeichnet, daß die Positioniereinrichtung aus einem elastischen Stopfen (11) besteht, der eine zentrale Öffnung aufweist, in der die Lichtleitersonde (10) mit Reibungswiderstand aufgenommen werden kann.

7. Kalibrierreflektorvorrichtung nach Anspruch 6 dadurch gekennzeichnet, daß der Stopfen (11) an seiner Außenseite mit Begrenzungsanschlägen zum Positionieren im Gehäuse (12) versehen ist.

## Claims

1. Calibration reflector device for an optical measuring system, which comprises an optical light-conductor probe (10), with
- a casing (12) which is open at one side;
- a reflecting device (14) with a predetermined reflection behaviour; and
- a positioning device (11) which arranges the front end of the light-conductor probe in the casing opposite the reflecting device, characterised by a transparent disc (13) which is disposed in front of the reflecting device (14) in such a manner that the front end of the light-conductor probe (10) is arranged by the positioning device (11) so as to abut the transparent disc (13).

2. Calibration reflector device according to Claim 1, characterised in that the reflecting device (14) consists of a reflecting layer in which reflecting particles are disposed.

3. Calibration reflector device according to Claim 2, characterised in that the reflecting layer is coated onto the transparent disc (13).

4. Calibration reflector device according to Claim 1, characterised in that the reflecting device is formed by reflecting particles being embedded in the front end of the casing (12).

5. Calibration reflector device according to Claim 1, characterised in that the surface (16) of the transparent disc (13) which borders on the reflecting device (14) is in the form of a coarse reflective boundary surface which is completely reflective in parts, and the reflecting device (14) consists of an opaque layer with a low refractive index.

6. Calibration reflector device according to one of the preceding Claims, characterised in that the positioning device consists of a resilient stopper (11), which comprises a central aperture, in which the light-conductor probe (10) can be accommodated with friction resistance.

7. Calibration reflector device according to Claim 6, characterised in that the stopper (11) has delimiting abutments on the outer side thereof for positioning in the casing (12).

## Revendications

1. Dispositif de calibrage à réflecteur pour un système de mesure optique avec une sonde optique à fibres optiques (10), comprenant
- un boîtier (12) ouvert sur un côté,
- un système réflecteur (14) avec un comportement de réflexion détermine,
- un système de positionnement (11), qui place l'extrémité avant de la sonde à fibres optiques face au système réflecteur dans le boîtier, caractérisé par une vitre transparente (13), qui est montée devant le système réflecteur (14), de telle sorte que le système de positionnement (11) place l'extrémité avant de la sonde à fibres optiques (10) contre la vitre transparente (13).

2. Dispositif de calibrage à réflecteur selon la revendication 1, caractérise en ce que le dispositif réflecteur (14) est formé d'une couche réfléchissante qui contient des particules réfléchissantes.

3. Dispositif de calibrage à réflecteur selon la revendication 2, caractérisé en ce que la couche réfléchissante est déposée sur la vitre transparente (13).

4. Dispositif de calibrage à réflecteur selon la revendication 1, caractérisé en ce que le système réflecteur est réalisé du fait que des particules réfléchissantes sont enrobées dans la paroi frontale du boîtier (12).

5. Dispositif de calibrage à réflecteur selon la revendication 1, caractérisé en ce que la surface (16), contiguë au système réflecteur (14), de la vitre transparente (13) forme une interface rugueuse, dont une partie est totalement réfléchissante, et le système réflecteur (14) est formé d'une couche opaque avec un faible indice de réfraction.

6. Dispositif de calibrage à réflecteur selon l'une quelconque des revendications précédentes, caractérisé en ce que le système de positionnement est formé d'un bouchon élastique (11) avec une ouverture centrale, dans laquelle on peut introduire avec une résistance due au frottement la sonde à fibres optiques (10).

7. Dispositif de calibrage à réflecteur selon la revendication 6, caractérisé en ce que les parois extérieures du bouchon (11) sont munies de butées de limitation pour son positionnement dans le boîtier (12).
